# EUROPEAN PATENT APPLICATION

(11) **EP 4 356 815 A1**
(43) Date of publication of application: **24.04.2024**
(21) Application number: 22825262.3
(22) Date of filing: 14.06.2022
(51) Int. Cl.: A61B 1/24, A61B 1/00, A61B 5/00, A61C 9/00, G01B 11/24, H02J 7/00, H02J 50/10, H02J 50/70

(54) **WIRELESS SCANNER AND CHARGING METHOD THEREOF**

(30) Priority: 15.06.2021 KR 20210077208
(71) Applicant: Medit Corp., Seoul 07207 (KR)
(72) Inventor: LEE, Dong Hoon, Seoul 02842 (KR); JANG, Kwang Jin, Seoul 02842 (KR)
(74) Representative: Klunker IP Patentanwälte PartG mbB
(86) International application number: PCT/KR2022/008358
(87) International publication number: WO 2022/265337

(57) **Abstract**

A wireless scanner according to the present invention comprises: a scanner body having a plurality of components arranged on the inside; and a scanner tip coupled to one end of the scanner body and having a passage therein through which light passes. The scanner body includes: an optical heating unit including at least one camera and a light projector; a power heating unit including a battery for supplying power to at least one among the camera and the light projector; and at least one charging module for charging the battery by generating a current in a reception coil formed inside the scanner body, the current in the reception coil being generated by a magnetic field generated from a transmission coil included in a cradle for holding the scanner body.

## Description

### [Technical Field]

The present disclosure relates to a wireless scanner and a method of charging the wireless scanner.

### [Background Art]

Recently, a three-dimensional scanner that is used to measure and analyze a narrow area, such as the inside of the mouth, is actively developed. The three-dimensional scanner needs to be manufactured to have a small size in order for a user (representatively a dentist) to easily scan the narrow area. Accordingly, a structure and a technology have been integrated into the three-dimensional scanner.

Furthermore, when the user obtains two-dimensional image data and/or three-dimensional data by using the three-dimensional scanner, a three-dimensional model that is generated based on the data is displayed on a display device. A conventional three-dimensional scanner needs to be disposed within a predetermined distance from an external device (e.g., a PC or a server) that is connected to the three-dimensional scanner in a wired way and that is separated from the three-dimensional scanner. Illustratively, if the three-dimensional scanner and the external device are connected by a communication cable having a length of about 1.5 m, a user can use the three-dimensional scanner only within a radius range of about 1.5 m from the external device. As the three-dimensional scanner is connected in a wired way, a degree of freedom of scanning of the user is greatly limited.

In order to solve a user's inconvenience according to a wired connection, a three-dimensional scanner using a wireless method is developed. A three-dimensional wireless scanner solves the problem with the three-dimensional scanner using the wired connection method to some extent through a battery, etc.

Meanwhile, the three-dimensional wireless scanner has an additional problem in that a discharged battery needs to be replaced with a new battery by detaching the discharged battery in order to charge the three-dimensional wireless scanner or the three-dimensional wireless scanner needs to be charged by using an external power source. In order to solve such a problem, the application of a contactless wireless charging method is actively discussed.

### [DISCLOSURE]

### [Technical Problem]

In order to solve the aforementioned problem, the present disclosure provides a wireless scanner including at least one charging module from which a current is generated by receiving a magnetic field that is generated from a transmitting coil included in a cradle.

Furthermore, the present disclosure provides a method of charging the wireless scanner, in which an operation of a part that performs a scan process is deactivated when wireless charging is performed through the charging module of the wireless scanner.

Technical objects of the present disclosure are not limited to the aforementioned objects, and the other objects not described above may be evidently understood from the following description by those skilled in the art.

### [Technical Solution]

In order to solve the aforementioned problem, a wireless scanner according to the present disclosure includes a scanner body having a plurality of parts disposed therein and a scanner tip coupled to one end of the scanner body and having a passage through which light passes therein. The scanner body includes an optical heating part including at least one camera and a light projector, a power heating part including a battery that supplies power to at least one of the camera and the light projector, and at least one charging module configured to charge the battery by a current that is generated in a receiving coil formed in the scanner body by a magnetic field generated from a transmitting coil included in a cradle on which the scanner body is held.

Furthermore, the charging module may be formed by being separated from the power heating part. An interval between the charging module and the optical heating part may be formed to be smaller than an interval between the charging module and the power heating part.

Furthermore, the charging module may be disposed within an internal space of the scanner body, and may be disposed on a lower side than the optical heating part and the power heating part.

Furthermore, the charging module may be formed in a shape corresponding to an external surface of the scanner body.

Furthermore, the wireless scanner may further include at least one slot that is upward depressed and formed at the bottom of the scanner body, wherein the charging module may be formed in a shape corresponding to at least one surface of the slot.

Furthermore, the scanner body may further include a magnetic unit that is formed at the bottom of the scanner body and that provides magnetism so that the scanner body is held on the cradle.

Furthermore, the scanner body may further include an electromagnetic shielding unit for preventing electromagnetic interference between the magnetic unit and the charging module.

Furthermore, the wireless scanner may further include a communication module for communicating with a physically separated device, wherein the communication module may be formed by being separated from a holding part of a user in the scanner body, and may be formed by being separated from the charging module by at least one of the optical heating part and the power heating part.

Furthermore, the wireless scanner may further include a controller configured to determine whether a current has been generated in the charging module, wherein the controller may deactivate an operation of the optical heating part when determining that the current has been generated in the charging module.

Meanwhile, a method of charging a wireless scanner according to the present disclosure includes holding a scanner including a charging module on a cradle that generates an electromagnetic force, generating a current in a charging module that is separated from the cradle at a predetermined interval by the cradle, and charging a battery that is electrically connected to the charging module and that supplies power to the scanner through the generated current.

Furthermore, the holding of the scanner may include holding the scanner so that the bottom of the scanner comes into contact with the cradle and the charging module is adjacent to the cradle.

Furthermore, the method may further include, before the generating of the current, determining, by a controller of the scanner, whether a current has been generated in the charging module, and controlling, by the controller, an operation of the scanner based on whether the current has been generated in the charging module.

Furthermore, the controlling of the operation of the scanner may include deactivating, by the controller, an operation of an optical heating part including at least one camera and a light projector when determining that the current has been generated in the charging module.

### [Advantageous Effects]

Through the wireless scanner and the method of charging the wireless scanner according to the present disclosure, there is an advantage in that a degree of freedom of scanning is increased because a user is free of a wired connection range in performing a scan process.

Furthermore, there are advantages in that the wireless scanner according to the present disclosure can be stably held because the wireless scanner is held on the cradle and the wireless scanner can be precisely charged with power although an external power source is not connected to the wireless scanner.

Furthermore, there is an advantage in that charging efficiency is maximized by a magnetic field generated from the transmitting coil because a shape of the charging module corresponds to a shape of the transmitting coil of the cradle.

Furthermore, there is an advantage in that the wireless scanner can be stably held in the cradle in a shape in which charging efficiency can be maximized because at least one slot is formed at the bottom of the scanner body and the charging module is formed in a shape corresponding to a shape of the slot.

Furthermore, there are advantages in that the wireless scanner is stably held on the cradle and charging efficiency is maximized because the magnetic unit is disposed under the scanner body and gravitation acts between the magnetic unit and the cradle magnetic unit formed in the cradle.

Furthermore, there is an advantage in that magnetic interference of the magnetic unit with the charging module is minimized because the electromagnetic shielding unit is formed between the charging module and the magnetic unit.

Furthermore, there are advantages in that unnecessary data are prevented from being obtained in a process of wireless charging being performed because the controller deactivates an operation of the optical heating part when a charging process is performed by the charging module and charging efficiency is maximized because unnecessary power consumption is minimized by deactivating an operation of the optical heating part during the charging of the wireless scanner.

### [Description of Drawings]

FIG. 1 is a perspective view of a wireless scanner according to the present disclosure.
FIG. 2 is a cross-sectional view of the wireless scanner according to the present disclosure.
FIG. 3 is for describing a process of the wireless scanner according to the present disclosure being held on a cradle and charged.
FIG. 4 is for describing a process of the wireless scanner according to the present disclosure being held on the cradle and charged and another shape of a charging module.
FIG. 5 is for describing a form in which at least one slot has been formed at the bottom of a scanner body in the wireless scanner according to the present disclosure.
FIG. 6 is for describing a process of the wireless scanner according to the present disclosure being held on the cradle and charged and still another shape of the charging module.
FIG. 7 is for describing a process of the wireless scanner according to the present disclosure being held by a magnetic unit in the wireless scanner.
FIG. 8 is for describing an electromagnetic shielding unit in the wireless scanner according to the present disclosure.
FIG. 9 is a schematic diagram of a construction of the wireless scanner according to the present disclosure.
FIG. 10 is for describing a process of charging being performed by a charging module in the wireless scanner according to the present disclosure.
FIG. 11 is a flowchart of a method of charging the wireless scanner according to the present disclosure.

### [Description of reference numerals]

| | | | |
|---|---|---|---|
| 1: | wireless scanner | 100: | scanner body |
| 110: | optical heating part | 120: | power heating part |
| 130: | charging module | 140: | communication module |
| 150: | slot | 160: | magnetic unit |
| 170: | electromagnetic shielding unit | 180: | controller |
| 190: | intake fan | | |
| 2: | cradle | 310: | cradle body |
| 320: | power generation module | 330: | cradle magnetic unit |
| S110: | step of being held on cradle | | |
| S120: | step of current being generated | | |
| S130: | step of determining whether current has been generated | | |
| S140: | step of controlling operation of scanner | | |
| S150: | step of charging battery | | |

### [Best Mode]

Hereinafter, some embodiments of the present disclosure will be described in detail with reference to exemplary drawings. In adding reference numerals to the components of each drawing, it should be noted that the same components have the same reference numerals as much as possible even if they are displayed in different drawings. Furthermore, in describing embodiments of the present disclosure, when it is determined that a detailed description of the related well-known configuration or function hinders understanding of an embodiment of the present disclosure, the detailed description thereof will be omitted.

Furthermore, in describing components of an embodiment of the present disclosure, terms, such as a first, a second, A, B, (a), and (b), may be used. Such terms are used only to distinguish one component from another component, and the essence, order, or sequence of a corresponding component is not limited by the terms. All terms used herein, including technical or scientific terms, have the same meanings as those commonly understood by a person having ordinary knowledge in the art to which an embodiment pertains, unless defined otherwise in the specification. Terms, such as those commonly used and defined in dictionaries, should be construed as having the same meanings as those in the context of a related technology, and are not construed as having ideal or excessively formal meanings unless explicitly defined otherwise in the specification

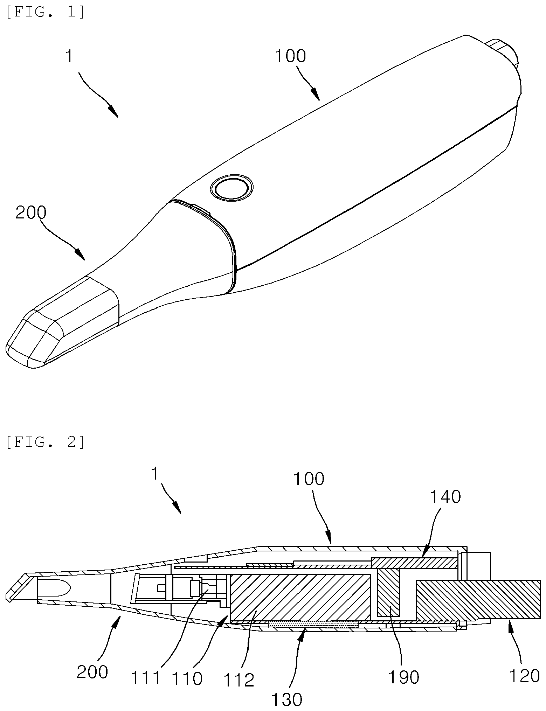

FIG. 1 is a perspective view of a wireless scanner 1 according to the present disclosure.

The wireless scanner 1 according to the present disclosure includes a scanner body 100 in which a plurality of major parts is disposed and a scanner tip 200.

Parts for scanning an object are embedded and disposed in the scanner body 100. Illustratively, a part for radiating light to the outside or receiving light that is received therein, a part for storing data, a part for communicating with the outside, a part for controlling an operation of the wireless scanner 1, a part for supplying power, etc. are embedded in the scanner body 100.

Furthermore, the scanner tip 200 has a shape having one side opened. A part for reflecting light may be embedded within the scanner tip. Through the scanner tip 200, light that is generated within the wireless scanner 1 may be radiated toward an object through an opening of the scanner tip 200. Light that is reflected by a surface of the object may be transmitted to the part for receiving the light through the opening of the scanner tip 200. That is, the scanner tip 200 may be coupled to one end of the scanner body 100, and may form a passage through which light passes therein.

The object is a target for obtaining a three-dimensional model by using the wireless scanner 1 according to the present disclosure. Illustratively, the object may be the inside of an actual mouth, including the teeth, gingiva, and a dental arch of a patient. Furthermore, the object may be an oral model that is obtained by the impression taking of the inside of the mouth of a patient. In general, an oral model may be fabricated by a plaster material.

Hereinafter, detailed components of the wireless scanner 1 according to the present disclosure are described.

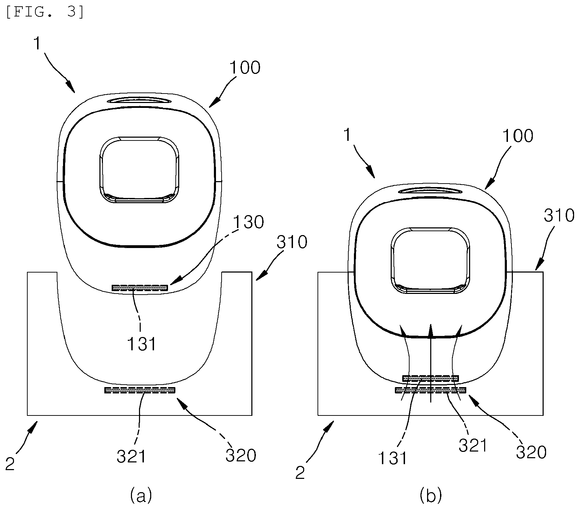

FIG. 2 is a cross-sectional view of the wireless scanner 1 according to the present disclosure. FIG. 3 is for describing a process of the wireless scanner 1 according to the present disclosure being held on a cradle 2 and charged. In particular, FIG. 3(a) is for describing the state in which the wireless scanner 1 has been separated from the cradle 2. FIG. 3(b) is for describing the state in which the wireless scanner 1 has been seated and held on the cradle 2 and charged.

Referring to FIG. 2, the wireless scanner 1 according to the present disclosure includes an optical heating part 110. The optical heating part 110 may be embedded in the scanner body 100, and may radiate light to the outside of the wireless scanner 1 or may receive light that is input to the wireless scanner 1. More specifically, the optical heating part 110 includes at least one camera 111 and a light projector 112. The camera 111 may receive light that is input to the wireless scanner 1 through the scanner tip 200. In this case, the light that is input to the wireless scanner 1 may include light that is reflected by a surface of an object. The camera 111 may be a mono camera or a stereo camera. Furthermore, the wireless scanner 1 may have one camera 111 or may have a plurality of cameras 111. Illustratively, if the wireless scanner 1 has a plurality of cameras 111, there is an advantage in that image data indicative of an object can be obtained more precisely.

An imaging sensor that is electrically connected to the camera 111 may generate image data on the basis of light that is received by the camera 111. Illustratively, the image data may be two-dimensional plane data. Furthermore, the imaging sensor may be a CCD sensor or a CMOS sensor. However, the imaging sensor is not limited to types that are listed in this specification, and may be any type of sensor for generating image data on the basis of light that is received by the camera 111.

Meanwhile, the light projector 112 may radiate light to the outside of the wireless scanner 1. Illustratively, the light projector 112 includes a light source (not illustrated) that generates predetermined light. The light source may generate a variable color or may generate a single color. If the light source generates a single color, the light projector 112 may include a plurality of light sources, and may implement various colors based on a combination of the light sources. Furthermore, the light projector 112 may further include a pattern generation part (not illustrated). The pattern generation part may radiate a structured light on a surface of an object so that the pattern generation part may generate a three-dimensional model on the basis of light that is input to the camera 111. Illustratively, the pattern generation part may be a pattern mask or may be a digital micromirror device (DMD).

Furthermore, the wireless scanner 1 according to the present disclosure may include a power heating part 120. The power heating part 120 may play a role of supplying power to a plurality of parts embedded in the wireless scanner 1 according to the present disclosure. Illustratively, the power heating part 120 may include a battery. The power heating part 120 may supply power to at least one of the camera 111 and the light projector 112. More specifically, the battery may supply power to the optical heating part 110 including the camera 111 and the light projector 112. The power heating part 120 may include a chargeable construction. Illustratively, the power heating part 120 may include a lithium-ion battery. However, the power heating part 120 does not need to essentially include the lithium-ion battery, and may include any type of battery which can be consistently used by being charged in wire and wireless manners without replacing the battery.

The wireless scanner 1 according to the present disclosure may further include an intake fan 190 within the scanner body 100. The intake fan 190 may suck the air outside the wireless scanner 1 into the scanner body 100 through a rotary motion, and may cool heated parts within the scanner body 100. Illustratively, the intake fan 190 may suck the air outside the wireless scanner 1 into the scanner body 100, and may induce an air flow path so that the sucked air is directed toward the optical heating part 110. The air that is moved by the intake fan 190 may forcedly cool the optical heating part 110. Heated air may be discharged through a rear surface of the scanner body 100 and/or the opening of the scanner tip 200. Meanwhile, the power heating part 120 does not have a high degree of heating compared to the optical heating part 110, so that a part of the power heating part 120 may be naturally cooled by being exposed to the outside of the scanner body 100.

Furthermore, the wireless scanner 1 according to the present disclosure may include a communication module 140. The communication module 140 may be disposed within the scanner body 100. The communication module 140 may communicate with a device that has been physically separated from the wireless scanner 1, that is, the outside. The communication module 140 may transmit, to the outside, image data that are obtained through the optical heating part 110, or may transmit a signal for indicating the state of the wireless scanner 1 to the outside. Furthermore, the communication module 140 may transmit a signal that is received from the outside of the communication module 140 to a controller 180 that is described later, so that the wireless scanner 1 according to the present disclosure can operate in response to the signal received from the outside. The communication module 140 may be a common construction capable of wireless communication, such as Wi-Fi, Bluetooth, Zigbee, or LoRa.

Meanwhile, the communication module 140 is formed by being separated from the holding part of a user, which is included in the scanner body 100. The communication module 140 may be disposed in a rear part of the scanner body 100 on the upper side thereof. The wireless scanner 1 according to the present disclosure may be formed in a shape that enables a user to grasp the wireless scanner 1 without hindering the transmission and reception of signals of the communication module 140. The communication module 140 is disposed at a specific location within the scanner body 100 so that a user can grasp a part that does not hinder the transmission and reception of signals of the communication module 140. Accordingly, the wireless scanner 1 according to the present disclosure has advantages in that the wireless scanner can smoothly communicate with a device that has been physically separated and a loss of image data that are received from the separated device can be minimized.

Hereinafter, a charging module 130, among detailed components of the wireless scanner 1 according to the present disclosure, is described.

Referring to FIGS. 2 and 3, the wireless scanner 1 according to the present disclosure may include at least one charging module 130 that charges a battery included in the power heating part 120. Illustratively, a current is generated in a receiving coil 131 that is formed within the scanner body 100 and that is included in the charging module 130 by a magnetic field generated from a transmitting coil 321 included in the cradle 2 on which the scanner body 100 is held. Accordingly, the charging module 130 can charge the battery.

More specifically, a power generation module 320 that is formed within a cradle body 310 having a U-shaped contact surface of the cradle 2 may generate a magnetic field by an external power source (not illustrated) electrically connected to the power generation module 320. The power generation module 320 may be formed to have a circle or a ring shape, but the present disclosure is not essentially limited thereto. The power generation module 320 may be formed in any shape capable of generating an electromagnetic field.

When the magnetic field is generated by the power generation module 320, the generated magnetic field affects the charging module 130 formed within the scanner body 100. More specifically, the charging module 130 receives the magnetic field transmitted by the power generation module 320, so that a current is generated in the receiving coil 131 of the charging module 130 by an electromagnetic induction principle. The generated current may move to the power heating part 120 electrically connected to the charging module 130, and charges the battery of the power heating part 120.

Hereinafter, an arrangement relation in the wireless scanner 1 of the charging module 130 is described.

As illustrated in FIG. 2, the charging module 130 may be formed by being separated from the power heating part 120. Illustratively, the charging module 130 may be formed between the optical heating part 110 and the power heating part 120 at a predetermined interval therefrom. The interval between the charging module 130 and the optical heating part 110 may be formed to be smaller than an interval between the charging module 130 and the power heating part 120. More specifically, the charging module 130 may be disposed to be adjacent to the bottom of the scanner body 100 within the scanner body 100. The optical heating part 110 may be disposed at a central part of the scanner body 100 within the scanner body 100. The power heating part 120 may be disposed at a rear part of the scanner body 100 within the scanner body 100.

As another meaning, the charging module 130 may be disposed in an internal space of the scanner body 100, and may be disposed on a lower side than the optical heating part 110 and the power heating part 120. Since the charging module 130 is disposed on a lower side than the optical heating part 110 and the power heating part 120, a distance from the power generation module 320 can be minimized when the charging module 130 is held on the cradle 2. Accordingly, there are advantages in that the charging module 130 can easily receive a magnetic field that is generated by the power generation module 320 and charging efficiency of the wireless scanner 1 is maximized.

Meanwhile, the communication module 140 may be formed by being separated from the charging module 130 by at least one of the optical heating part 110 and the power heating part 120. The charging module 130 is a part that receives a magnetic field attributable to electromagnetic induction, and the communication module 140 is formed by being separated from the charging module 130. Accordingly, when the communication module 140 communicates with the outside, interference attributable to electromagnetic induction, which may affect the communication, is prevented.

Hereinafter, a shape of the charging module 130 is more specifically described.

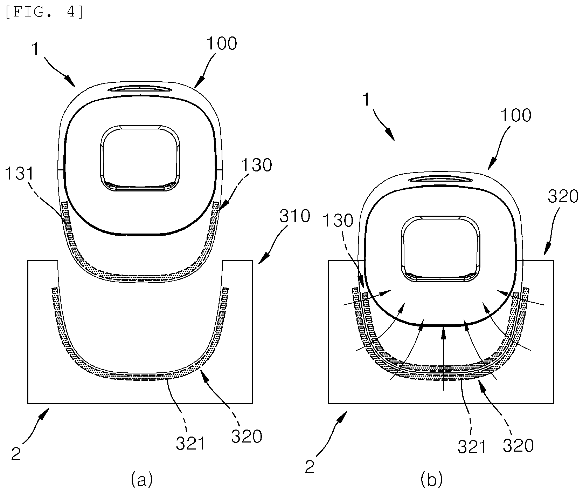

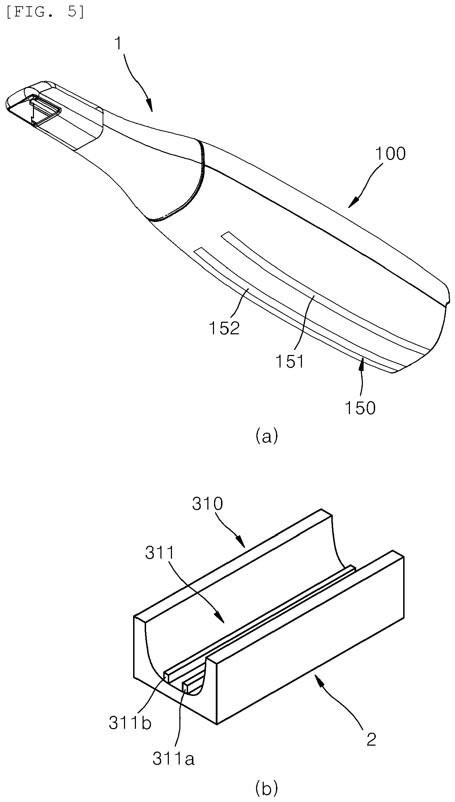

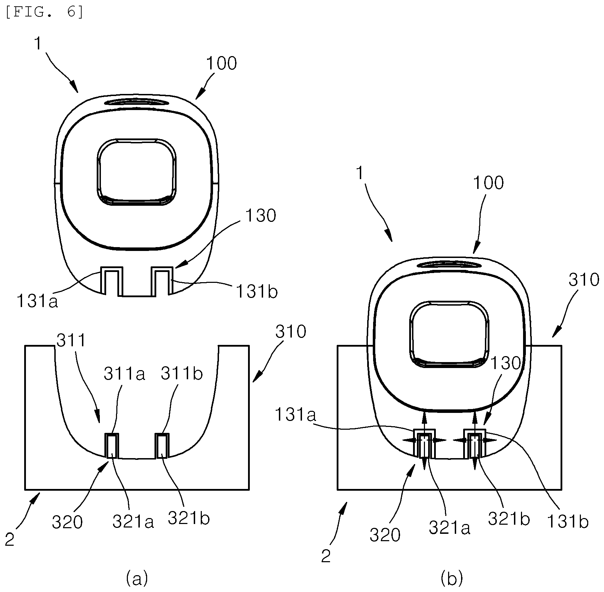

FIG. 4 is for describing a process of the wireless scanner 1 according to the present disclosure being held on the cradle 2 and charged and another shape of the charging module 130. FIG. 5 is for describing a form in which at least one slot 150 has been formed at the bottom of the scanner body 100 in the wireless scanner 1 according to the present disclosure. FIG. 6 is for describing a process of the wireless scanner 1 according to the present disclosure being held on the cradle 2 and charged and still another shape of the charging module 130. More specifically, FIG. 4(a) is for describing the state in which the wireless scanner 1 has been separated from the cradle 2. FIG. 4(b) is for describing the state in which the wireless scanner 1 has been seated and held on the cradle 2 and charged. FIG. 5(a) is for describing the bottom of the wireless scanner 1 having the at least one slot 150. FIG. 5(b) is for describing the cradle 2 corresponding to the wireless scanner 1 having the slot 150. FIG. 6(a) is for describing the state in which the wireless scanner 1 has been separated from the cradle 2. FIG. 6(b) is for describing the state in which the wireless scanner 1 has been seated and held on the cradle 2 and charged.

As any one example, referring to FIG. 3, the charging module 130 may be formed in a plate shape having a rectangular parallelepiped. Furthermore, the power generation module 320 of the cradle 2 corresponding to the charging module 130 may also be formed in a plate shape having a rectangular parallelepiped. The charging module 130 and the power generation module 320 may be formed to face each other in parallel. Accordingly, there are advantages in that electromagnetic induction between the power generation module 320 and the charging module 130 is maximized and charging efficiency of the battery is maximized because the charging module 130 and the power generation module 320 face each other in parallel at a minimized interval when the wireless scanner 1 is seated and held on the cradle 2 as illustrated in FIG. 3(b).

As another example, referring to FIG. 4, the charging module 130 may be formed in a shape corresponding to an external surface of the scanner body 100. Illustratively, the charging module 130 may have a streamlined (more specifically, U-shaped) structure so that the charging module 130 corresponds to the bottom of the scanner body 100. The power generation module 320 of the cradle 2 may also have a streamlined (U-shaped) structure, that is, a shape corresponding to the charging module 130, so that the power generation module 320 corresponds to the charging module 130. Accordingly, as illustrated in FIG. 4(b), when the wireless scanner 1 is seated and held on the cradle 2, the charging module 130 is formed to face the power generation module 320 in parallel, and the interval between the charging module 130 and the power generation module 320 is minimized. Accordingly, there is an advantage in that charging efficiency of the wireless scanner 1 is maximized.

As another example, referring to FIGS. 5 and 6, the wireless scanner 1 according to the present disclosure may include at least one slot 150 that is upward depressed and formed at the bottom of the scanner body 100. The slot 150 may be formed in the length direction of the wireless scanner 1, but the present disclosure is not essentially limited thereto. Illustratively, a first slot 151 and second slot 152 of the slot 150 may be formed in parallel as a pair. Furthermore, the cradle 2 may include a cradle protrusion part 311 having a shape corresponding to the slot 150 in order to accommodate the wireless scanner 1. The cradle protrusion part 311 may be formed to protrude at a predetermined height from a contact surface of the cradle protrusion part 311 on which the wireless scanner 1 is held toward a direction in which the wireless scanner 1 is held. If the wireless scanner 1 includes the first slot 151 and the second slot 152, the cradle protrusion part 311 may include a first cradle protrusion part 311a and a second cradle protrusion part 311b so that the first cradle protrusion part 311a and the second cradle protrusion part 311b correspond to the first slot 151 and the second slot 152.

When the wireless scanner 1 according to the present disclosure is seated and held on the cradle 2, the slot 150 accommodates the cradle protrusion part 311. Accordingly, the wireless scanner 1 may be disposed at its regular position without being rotated with respect to the cradle 2. Accordingly, the charging module 130 and the power generation module 320 may be disposed at a location having optimal charging efficiency.

Meanwhile, if the wireless scanner 1 includes the slot 150, the charging module 130 may be formed in a shape corresponding to at least one surface of the slot. Illustratively, the charging module 130 may include a first charging coil 131a formed in a shape corresponding to the first slot 151 and a second charging coil 131b formed in a shape corresponding to the second slot 152. The first charging coil 131a and the second charging coil 131b may have a cross-sectional shape that surrounds the slot 150 within the scanner body 100 like a Hangeul consonant " 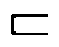 " (or a channel shape). The power generation module 320 also includes a first power generation coil 321a corresponding to the first cradle protrusion part 311a and a second power generation coil 321b corresponding to the second cradle protrusion part 311b. The first power generation coil 321a and the second power generation coil 321b are surrounded by the first charging coil 131a and the second charging coil 131b, respectively, when the slot 150 is accommodated in the cradle protrusion part 311. Accordingly, there is an advantage in that charging efficiency of the wireless scanner 1 is maximized because the charging module 130 can effectively receive a magnetic field transmitted by the power generation module 320.

Hereinafter, a construction of a magnetic unit 160 for enabling the wireless scanner 1 according to the present disclosure to be disposed in the cradle 2 at its regular position in is described.

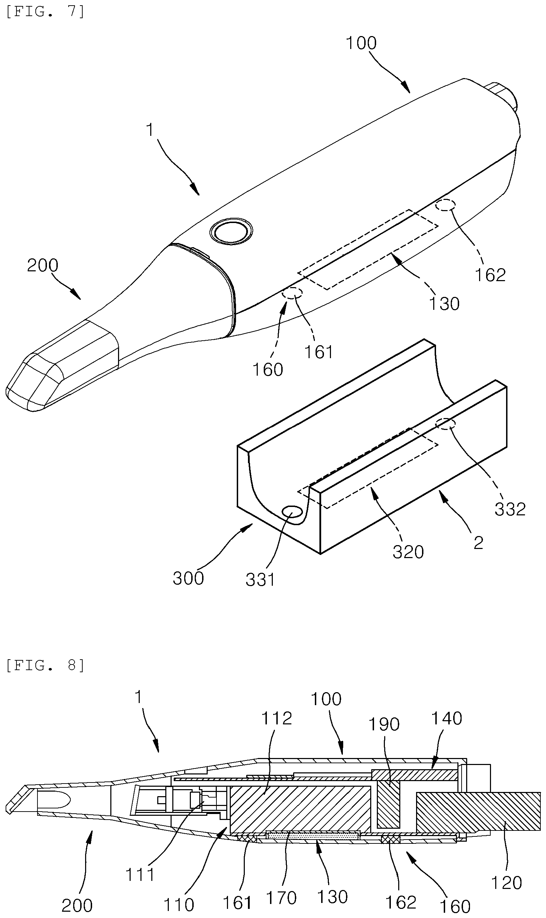

FIG. 7 is for describing a process of the wireless scanner 1 according to the present disclosure being held by the magnetic unit 160 in the wireless scanner. FIG. 8 is for describing an electromagnetic shielding unit 170 in the wireless scanner 1 according to the present disclosure.

Referring to both FIGS. 7 and 8, in the wireless scanner 1 according to the present disclosure, the scanner body 100 may further include at least one magnetic unit 160 that provides magnetism so that the scanner body 100 can be stably held on the cradle 2. Illustratively, the magnetic unit 160 may be formed at the bottom of the scanner body 100. The magnetic unit 160 may be embedded in the scanner body 100 like other internal parts thereof, and may be formed in an external surface of the scanner body 100. A first magnetic unit 161 and second magnetic unit 162 of the magnetic unit 160 may be formed to face each other on the basis of a location where the charging module 130 has been formed.

Furthermore, the cradle 2 may include at least one cradle magnetic unit 330 on which gravitation acts along with the magnetic unit 160 at a location corresponding to the magnetic unit 160. If the magnetic unit 160 of the wireless scanner 1 includes the first magnetic unit 161 and the second magnetic unit 162, the cradle magnetic unit 330 may also include a first cradle magnetic unit 331 and a second cradle magnetic unit 332 corresponding to the first magnetic unit 161 and the second magnetic unit 162, respectively. When the wireless scanner 1 according to the present disclosure is held on the cradle 2, gravitation mutually acts between the first magnetic unit 161 and the first cradle magnetic unit 331, and gravitation mutually acts between the second magnetic unit 162 and the second cradle magnetic unit 332. Accordingly, the wireless scanner 1 according to the present disclosure may be disposed at its regular position at a specific location of the cradle 2. The wireless scanner 1 may be held on the cradle 2 so that the charging module 130 and the power generation module 320 have a location relation having optimal charging efficiency.

Meanwhile, referring to FIG. 8, interference may occur in an electromagnetic induction process between the power generation module 320 and the charging module 130 due to magnetism that is generated by the magnetic unit 160. That is, although the wireless scanner 1 is stably held on the cradle 2 by the magnetic unit 160, there is a possibility that charging efficiency may be reduced due to the magnetism provided by the magnetic unit 160. Accordingly, the wireless scanner 1 according to the present disclosure further includes the electromagnetic shielding unit 170 for preventing electromagnetic interference between the magnetic unit 160 and the charging module 130 in the scanner body 100. The electromagnetic shielding unit 170 may be formed in a shape that covers at least one surface of the charging module 130. Illustratively, the electromagnetic shielding unit 170 may be formed between the charging module 130 and the magnetic unit 160. Additionally, the electromagnetic shielding unit 170 may be formed between the charging module 130 and the optical heating part 110 and/or the power heating part 120 in order to minimize the influence of a magnetic field that is applied to the optical heating part 110 and the power heating part 120. That is, the electromagnetic shielding unit 170 may be formed in a shape that covers two sides, four sides, or the top, and four sides of the charging module 130 except the bottom of the scanner body 100 in which the wireless scanner 1 is held on the cradle 2. The electromagnetic shielding unit 170 may have a shape of a case made of a metal material, such as aluminum or copper, may use a metal thin film, and may use any material capable of minimizing magnetic interference of the magnetic unit 160, which may be applied to the charging module 130.

Hereinafter, a construction of the controller 180 that controls an operation of the wireless scanner 1 according to the present disclosure is described.

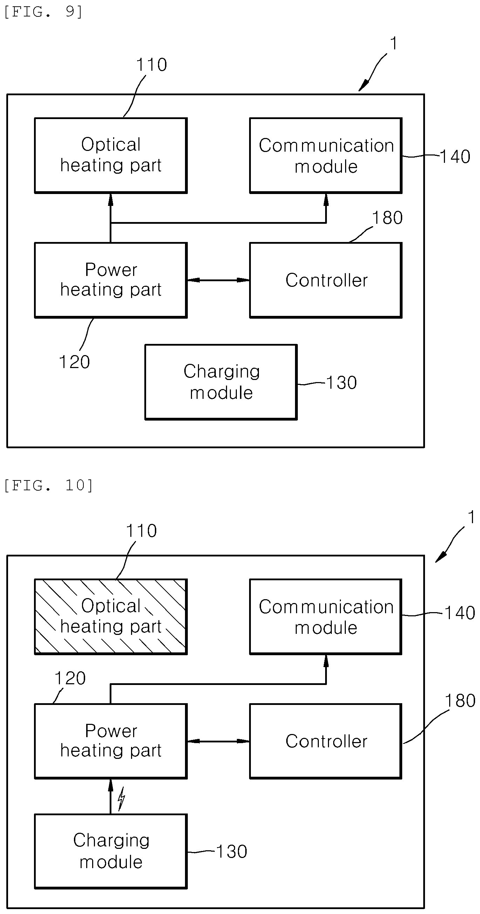

FIG. 9 is a schematic diagram of a construction of the wireless scanner according to the present disclosure. FIG. 10 is for describing a process of charging being performed by the charging module in the wireless scanner according to the present disclosure.

Referring to FIG. 9, the wireless scanner 1 according to the present disclosure further includes the controller 180. The controller 180 controls an operation of the wireless scanner 1 according to the present disclosure. Illustratively, the controller 180 may control the optical heating part 110 to operate and obtain image data, and may control the communication module 140 to transmit stored image data to the outside. In order to perform such operation functions, the controller 180 may include a microprocessor.

Furthermore, the controller 180 determines whether a current has been generated in the charging module 130, and controls operations of at least some components of the wireless scanner 1 based on whether a current has been generated in the charging module 130. As illustrated in FIG. 9, if the wireless scanner 1 is not held on the cradle 2 or a magnetic field is not generated by the power generation module 320 because power is not supplied to the cradle 2 although the wireless scanner 1 is held on the cradle 2, the wireless scanner 1 according to the present disclosure operates normally, and the battery of the power heating part 120 can supply power to the optical heating part 110 and the communication module 140. Accordingly, a user may perform a scan operation by using the wireless scanner 1 according to the present disclosure.

However, if the wireless scanner 1 is held on the cradle 2 and wireless charging is performed, the wireless scanner 1 may charge the battery of the power heating part 120 without scanning an object, and may communicate with the outside through the communication module 140. The optical heating part 110 may not operate during the wireless charging of the wireless scanner 1 because it is inefficient to perform a scan process in the state in which the wireless scanner 1 has been held on the cradle 2.

More specifically, as illustrated in FIG. 10, when determining that a current has been generated in the charging module 130 due to electromagnetic induction, the controller 180 may deactivate an operation of the optical heating part 110. That is, when the wireless scanner 1 is held on the cradle 2 and starts to be charged, the controller 180 may limit the acquisition of image data by a scan process by deactivating an operation of the optical heating part 110. That is, if the wireless scanner 1 is held on the cradle 2 and charging is performed although a user does not directly terminate the scan process of the wireless scanner 1, the supply of power to the optical heating part 110 may be automatically blocked and the acquisition of the image data may be limited. While the wireless scanner 1 is charged, the wireless scanner 1 may transmit, to the outside, image data obtained by a scan process prior to the charging through the communication module 140, and may display the charging state of the wireless scanner 1 on a display device (not illustrated) that is physically separated from the wireless scanner 1 by transmitting the charging state to the outside. Illustratively, the display device may include a monitor, a tablet device, etc., which can visually notify a user of the state of the wireless scanner 1 according to the present disclosure.

When a current is generated in the charging module 130, the controller 180 deactivates an operation of the optical heating part 110. A user directly holds the wireless scanner 1 on the cradle 2 without stopping a scan process of the wireless scanner 1 so that the scan operation is automatically stopped. Accordingly, there are advantages in that a user's inconvenience is minimized, the waste of unnecessary resources is minimized by limiting the acquisition of image data during charging, and charging efficiency of the wireless scanner 1 through the charging module 130 is maximized by minimizing power consumption of the optical heating part 110 that is deactivated during charging.

Hereinafter, a method of controlling the wireless scanner according to the present disclosure is described in detail. In describing the method of controlling the wireless scanner, a description that is redundant with that of the wireless scanner 1 is provided in short or omitted.

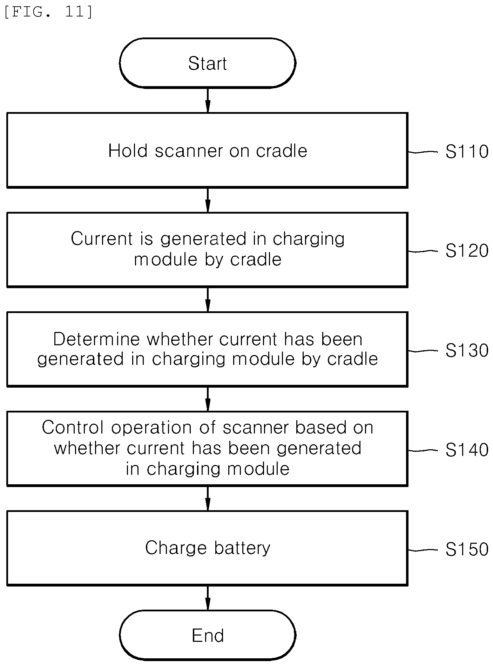

FIG. 11 is a flowchart of a method of charging the wireless scanner according to the present disclosure.

Referring to FIG. 11, the method of charging the wireless scanner according to the present disclosure includes step S110 of holding the scanner (i.e., the wireless scanner according to the present disclosure) including the charging module on the cradle that generate an electromagnetic force. A user may obtain image data indicative of an object by using the scanner, and may hold the scanner on the cradle when the acquisition of the image data is completed or for a short break. In this case, the user may hold the scanner so that the bottom of the scanner comes into contact with the cradle and the charging module is adjacent to the cradle. For example, the cradle may have a contact surface having a shape corresponding to an external surface of the scanner body. The bottom of the scanner body may be seated on the contact surface of the cradle. Accordingly, the charging module formed at the bottom of the scanner may be disposed to be adjacent to the power generation module embedded in the cradle body, and charging efficiency of the wireless scanner is maximized. The cradle is connected to an external power source. A magnetic field is generated by the power generation module embedded in the cradle body.

Meanwhile, the method of charging the wireless scanner according to the present disclosure includes step S120 of a current being generated in the charging module by the cradle. In step S120 of the current being generated, the current may be generated in the charging module included in the scanner according to an electromagnetic induction principle by the influence of a magnetic field that is generated by the power generation module.

The current that is generated in the charging module may move to the power heating part electrically connected to the charging module, and may charge (S150) the battery that supplies power to the scanner. The battery may supply power for operations of the optical heating part, the communication module, the controller, etc. That is, the charging module may provide the battery of the power heating part with the current that is induced by an electromagnetic force generated by the power generation module. The battery of the power heating part may be charged by the charging module.

Meanwhile, the method of charging the wireless scanner according to the present disclosure may further include step S130 of determining, by the controller of the scanner, whether a current has been generated in the charging module before the battery is charged. Illustratively, the controller may determine whether a current has been generated in the charging module based on at least one of the amount of current, the amount of power, a change in the amount of current, and a change in the amount of power in the charging module. The controller may control operations of at least some of internal components of the scanner based on whether a current has been generated in the charging module.

When the controller determines whether a current has been generated in the charging module, the controller may control (S140) an operation of the scanner based on whether the current has been generated in the charging module. Illustratively, in step S140 of controlling an operation of the scanner, the controller may deactivate an operation of the optical heating part including the at least one camera and the light projector when determining that a current has been generated in the charging module. That is, when the wireless scanner according to the present disclosure is held on the cradle and wireless charging is performed, the scanner may limit the acquisition of image data by the optical heating part so that a scan process is not performed. Accordingly, by using the method of charging the wireless scanner according to the present disclosure, the waste of unnecessary resources is prevented and power consumption of the scanner is minimized because an operation of the optical heating part is deactivated by only a user's behavior of holding, on the cradle, the wireless scanner that is used by the user. There is also an advantage in that charging efficiency of the wireless scanner through the charging module is maximized because power consumption of the scanner is minimized.

The above description is merely a description of the technical spirit of the present disclosure, and those skilled in the art may change and modify the present disclosure in various ways without departing from the essential characteristic of the present disclosure.

Accordingly, the embodiments described in the present disclosure should not be construed as limiting the technical spirit of the present disclosure, but should be construed as describing the technical spirit of the present disclosure. The technical spirit of the present disclosure is not restricted by the embodiments. The range of protection of the present disclosure should be construed based on the following claims, and all of technical spirits within an equivalent range of the present disclosure should be construed as being included in the scope of rights of the present disclosure.

### [Industrial Applicability]

The present disclosure provides the wireless scanner including the charging module that charges the battery that provides power for operations of the optical heating part and the power heating part and the method of charging the wireless scanner, in which the wireless scanner is held on the cradle and charged.

## Claims

1. A wireless scanner comprising:
a scanner body having a plurality of parts disposed therein; and
a scanner tip coupled to one end of the scanner body and having a passage through which light passes therein,
wherein the scanner body comprises:
an optical heating part comprising at least one camera and a light projector;
a power heating part comprising a battery that supplies power to at least one of the camera and the light projector; and
at least one charging module configured to charge the battery by a current that is generated in a receiving coil formed in the scanner body by a magnetic field generated from a transmitting coil included in a cradle on which the scanner body is held.

2. The wireless scanner of claim 1, wherein:
the charging module is formed by being separated from the power heating part, and
an interval between the charging module and the optical heating part is formed to be smaller than an interval between the charging module and the power heating part.

3. The wireless scanner of claim 1, wherein the charging module is disposed within an internal space of the scanner body and is disposed on a lower side than the optical heating part and the power heating part.

4. The wireless scanner of claim 1, wherein the charging module is formed in a shape corresponding to an external surface of the scanner body.

5. The wireless scanner of claim 1, further comprising at least one slot that is upward depressed and formed at a bottom of the scanner body, wherein the charging module is formed in a shape corresponding to at least one surface of the slot.

6. The wireless scanner of claim 1, wherein the scanner body further comprises a magnetic unit that is formed at a bottom of the scanner body and that provides magnetism so that the scanner body is held on the cradle.

7. The wireless scanner of claim 6, wherein the scanner body further comprises an electromagnetic shielding unit for preventing electromagnetic interference between the magnetic unit and the charging module.

8. The wireless scanner of claim 1, further comprising a communication module for communicating with a physically separated device, wherein the communication module is formed by being separated from a holding part of a user in the scanner body, and is formed by being separated from the charging module by at least one of the optical heating part and the power heating part.

9. The wireless scanner of claim 1, further comprising a controller configured to determine whether a current has been generated in the charging module, wherein the controller deactivates an operation of the optical heating part when determining that the current has been generated in the charging module.

10. A method of charging a wireless scanner, comprising:
holding a scanner comprising a charging module on a cradle that generates an electromagnetic force;
generating a current in a charging module that is separated from the cradle at a predetermined interval by the cradle; and
charging a battery that is electrically connected to the charging module and that supplies power to the scanner through the generated current.

11. The method of claim 10, wherein the holding of the scanner comprises holding the scanner so that a bottom of the scanner comes into contact with the cradle and the charging module is adjacent to the cradle.

12. The method of claim 10, further comprising:
before the generating of the current,
determining, by a controller of the scanner, whether a current has been generated in the charging module; and
controlling, by the controller, an operation of the scanner based on whether the current has been generated in the charging module.

13. The method of claim 12, wherein the controlling of the operation of the scanner comprises deactivating, by the controller, an operation of an optical heating part comprising at least one camera and a light projector when determining that the current has been generated in the charging module.
